# EUROPEAN PATENT SPECIFICATION

(11) **EP 4 516 210 B1**
(45) Date of publication and mention of the grant of the patent: **15.04.2026**
(21) Application number: 24196419.6
(22) Date of filing: 26.08.2024
(51) Int. Cl.: A61B 5/00

(54) **IMPLANT HEALTH SCORE**
IMPLANTAT-GESUNDHEITSSCORE
SCORE DE SANTÉ DES IMPLANTS

(30) Priority: 31.08.2023 US 202363535623 P
(43) Date of publication of application: 05.03.2025
(73) Proprietor: Howmedica Osteonics Corp., Mahwah, NJ 07430 (US)
(72) Inventor: MEYER, Andrew, Plantation, 33317 (US); VERSTRAETE, Matthias, 4861PK Chaam (NL); ANTUNES, Ricardo, Largs, KA30 9PD (GB)
(74) Representative: Keller Schneider Patentanwaltsgesellschaft mbH

(56) References cited:
- EP-A1- 4 042 978
- US-A1- 2006 047 283
- US-A1- 2017 296 118
- US-A1- 2023 255 794
- QIBLAWEY YAZAN ET AL: "Instrumented Hip Implant: A Review", IEEE SENSORS JOURNAL, IEEE, USA, vol. 21, no. 6, 16 December 2020 (2020-12-16), pages 7179 - 7194, XP011839315, ISSN: 1530-437X, [retrieved on 20210217], DOI: 10.1109/JSEN.2020.3045317

## Description

### CROSS-REFERENCE TO RELATED APPLICATIONS

This application claims the benefit of the filing date of United States Provisional Patent Application No. 63/535,623 filed August 31, 2023.

### FIELD OF INVENTION

The present disclosure relates to systems and methods for tracking implant performance, and particularly to systems and methods for tracking joint implant performance.

### BACKGROUND OF THE INVENTION

It is essential to monitor the recovery of a patient after a joint replacement surgery for a safe and successful rehabilitation. An important part of this monitoring is verifying the performance of an implant and detecting any complications such as dislocation, wear and tear, looseness, subsidence, and breakage. For example, a tibial insert which is typically composed of polyethylene and implanted during a total knee arthroscopy can be damaged if it receives excessive loading and thus, causing premature failure. Additionally, if an infection or inflammation is detected at the implantation point, early detection and correction of the problem is essential to prevent the implant from deteriorating. Furthermore, gathering data regarding the postoperative range of motion of the new joint implants and balancing the load correctly can provide helpful information to manage the patient's recovery and determine an appropriate replacement for the implant when necessary.

With the development of implantable sensors which are capable of measuring parameters of joint replacements such as kinematics, temperature, and wear depth, various potential failure modes for joint replacement may be identified. Currently, these implanted sensors which collect data can be utilized. However, creating a reliable way of using sensor data to make clinical decisions remains difficult due to the lack of established protocols.

Developing metrics based on the sensor measurements which offer insightful information about the patient's recovery progress, the state of the implant, and the likelihood of further failure are necessary in order to interpret the new data and provide assistance to clinicians when making critical clinical decisions. Relevant prior art is disclosed in US2023/255794 A1 and EP 4 0429 78 A1.

Therefore, there exists a need for systems and methods for tracking implant performance.

### BRIEF SUMMARY OF THE INVENTION

Disclosed herein are systems and methods for evaluating implant condition and associated patient condition.

According to the present invention there is provided a computer implemented method for determining an implant condition, the method comprising the steps of:
receiving implant vibration data including vibration measurement from an implant coupled to a bone of a patient, the implant vibration data being generated by a sensor associated with the implant;
receiving patient reported data including pain level from the patient, and
generating, using an algorithm, an implant loosening score from the implant vibration data and the patient reported data,
wherein the implant loosening score increases when the vibration measurement and the patient reported pain level increase, and wherein the implant loosening score is related to movement of implant with respect to the bone.

Continuing in accordance with this aspect, the step of generating the implant loosening score may include generating the implant loosening score based on pre-surgery data of the patient.

Continuing in accordance with this aspect, may include generating the implant loosening score based on a database of implant loosening scores of multiple patients.

Continuing in accordance with this aspect, the implant may be a joint implant. The sensor may be any of an inertial measurement unit sensor, accelerometer, gyroscope, Hall sensor, pH sensor, a temperature sensor and a pressure sensor operatively coupled to a processor of the joint implant. The implant may include a plurality of sensors.

Continuing in accordance with this aspect, the joint implant may be a knee joint implant. The patient reported data may include pain level associated with the knee joint implant.

Continuing in accordance with this aspect, the implant loosening score may be a single numerical value.

Continuing in accordance with this aspect, the implant loosening score may include multiple numerical values.

Continuing in accordance with this aspect, the implant loosening score may be generated as a graphical plot.

In accordance with another aspect of the present disclosure, a method for determining an implant condition is provided. A method according to this aspect may include the steps of receiving joint range of motion data from an implant coupled to a joint of a patient, receiving patient reported data from the patient, and generating an implant infection potential score from the joint range of motion data and the patient reported data. The joint range of motion may be generated by a sensor associated with the implant. The implant infection potential score may be related to infection of the implant or the joint of the patient.

Continuing in accordance with this aspect, the method may further include a step of receiving input from a healthcare professional and generating the implant infection potential score based on the input.

Continuing in accordance with this aspect, the joint may be any of a knee joint, shoulder joint, and ankle joint.

Continuing in accordance with this aspect, the joint may be a knee joint and the joint range of motion data may be related to flexion and extension of the knee joint.

Continuing in accordance with this aspect, the patient reported data may include pain level at the knee joint.

Continuing in accordance with this aspect, the patient reported data may include knee joint swelling data.

Continuing in accordance with this aspect, the method may further include the steps of receiving data from a second sensor of the implant and generating the implant infection potential score from the data from the second sensor. The second sensor may be any of pH sensor, temperature, and pressure sensor.

Continuing in accordance with this aspect, the implant infection potential score may be any of a single numerical value, multiple numerical values or a graphical plot.

### BRIEF DESCRIPTION OF THE DRAWINGS

A more complete appreciation of the subject matter of the present disclosure and the various advantages thereof can be realized by reference to the following detailed description, in which reference is made to the following accompanying drawings:
FIG. 1 is a schematic view of a system for evaluating an implant health score according to an embodiment of the present disclosure;
FIG. 2 is a schematic view of an implant health score according to an embodiment of the present disclosure;
FIG. 3 is a schematic view of an implant health score according to another embodiment of the present disclosure;
FIG. 4 is a schematic view of an implant health score according to another embodiment of the present disclosure;
FIG. 5 is a schematic view of an implant health score according to another embodiment of the present disclosure;
FIG. 6 is a schematic view of an implant health score according to another embodiment of the present disclosure;
FIG. 7 is a schematic view of an implant health score according to another embodiment of the present disclosure;
FIG. 8 is a plot of an implant health score according to an embodiment of the present disclosure, and
FIG. 9 is a plot of an implant health score according to another embodiment of the present disclosure.

### DETAILED DESCRIPTION

Reference will now be made in detail to the various embodiments of the present disclosure illustrated in the accompanying drawings. The term "a," as used in the specification, means "at least one." The terminology includes the words above specifically mentioned, derivatives thereof, and words of similar import. Although at least two variations are described herein, other variations may include aspects described herein combined in any suitable manner having combinations of all or some of the aspects described.

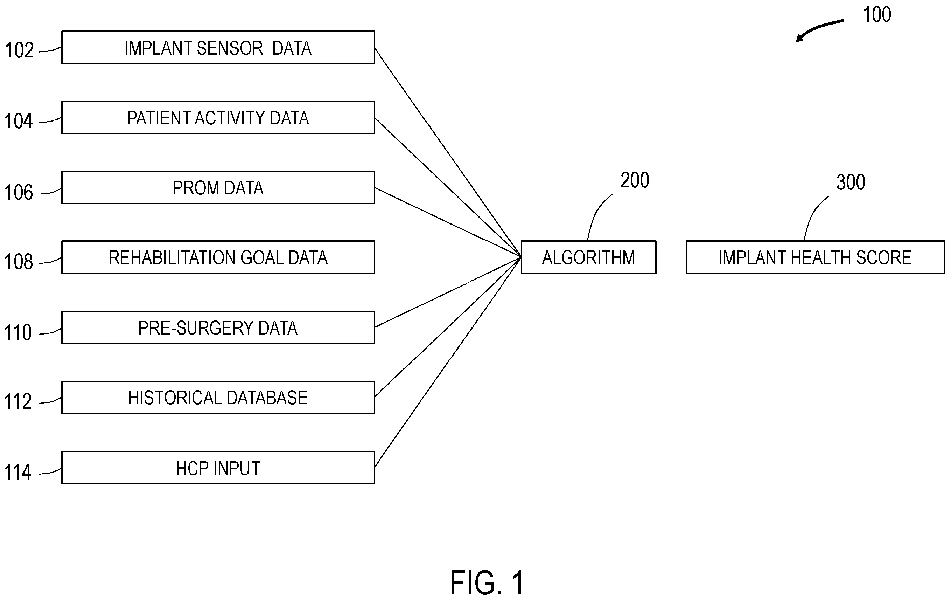

FIG. 1 is a schematic view of a system 100 for evaluating an implant health score 300 according to an embodiment of the present disclosure. The term "implant health score" should be understood to include a patient's recovery progress, implant condition, likelihood of implant failure, implant-related infection status, etc. Implant health scores disclosed herein can include any single one of these metrics or any combinations thereof. These metrics can be used in conjunction with one another to assess the overall health of the implant and provide an accurate analysis of the implant's performance and related patient condition. Implant health scores can be quantified using single or multiple numerical values or be demonstrated graphically in order to provide further detail. These graphical formats can include charts, histograms, pie charts, and other visual aids that help to illustrate the patient's health and performance of implants. Such visual aids may provide a more comprehensive understanding of their data and may allow for more accurate conclusions to be readily drawn from the implant health scores. System 100 can receive various input parameters from a multiple of sources as shown in FIG. 1. These inputs can include implant sensor data 102, patient activity data 104, patient-reported outcome measures ("PROMs") 106, rehabilitation goal data 108, pre-surgery data 110, historical database 112, healthcare professional (HCP) input 114, etc. While multiple inputs are shown in FIG. 1, one or more of these inputs can be used in other embodiments of the present disclosure.

One or more of these inputs are analyzed by an algorithm 200 which then provides implant health score 300. Algorithm 200 can include statistical analysis, decision trees, neural networks, Bayesian networks, etc. Statistical analysis can include regression analysis, analysis of variance, chi-square tests, time series analysis, cluster analysis, etc. Decisions trees can be used to visualize the outcomes of the inputs and implant health score, while neural networks can be used to recognize patterns and predict future implant health scores. Bayesian networks can use probability to compare input data points to determine possible implant health score outcomes. Algorithm 200 can be located on a server, smartphone, smart devices, implant, cloud, etc. Patients and HCPs can use algorithm 200 to analyze data to make better health decisions. Creating comprehensive metrics from these inputs using algorithm 200 can help HCPs and patients more accurately interpret the data and make informed decisions regarding the recovery process, implant health, and risk of device failure. By establishing these simple, yet precise, metrics, HCPs can easily recognize patterns and trends, leading to improved medical care and better long-term outcomes.

Implant sensor data 102 can include any data received from or derived from sensors located in or on an implant or trial. Any type of implant or trial with one or more sensors capable of outputting sensor information related to implant or trial condition or related patient condition can be transmitted to algorithm 200. Example of implants, including joint implants and the associated sensors and sensor data, are disclosed in U.S. Patent Publication No. 2023/0255794. Examples of implants with sensors can includes knee joint implants, hip implants, shoulder implants, intramedullary nails, bone plates, interference screws, external fixation devices, etc. Sensor associated with these implants can in include load sensors, temperature sensors, pH sensors, pressure sensors, Hall sensors, magnetic sensors, accelerometers, gyroscopes, inertial measurement units, etc.

Patient activity data 104 can include various forms of data points such as joint motion range, for example the extension and flexion of the patient's joints, the number of steps that the patient walks, the speed of their walking (from the steps per minute), the stride pattern that the patient walks with (also known as their gait), heart rate, oxygen saturation, etc. Patient activity data 104 can be determined by an implanted sensor such as knee joint motion range determined by a knee joint sensor with a Hall sensor and/or an external wearable sensor such as oxygen saturation level measured by a pulse oximeter sensor.

PROM data 106 can include patient-evaluated pain rating, range of motion assessment, muscle strength assessment, quality of life questionnaires, patient satisfaction, physical activity levels, weight bearing abilities, etc. Rehabilitation goal data 108 can include desired joint range of motion, pain levels, balance and strength levels, activity level, etc. A patient's pre-surgery data 110 can include a variety of information such as pre-surgery joint range of motion data, physical activity level assessments, pain level measurements, muscle strength testing, and other pertinent clinical information that can be relayed to algorithm 200. This information can be used to identify the patient's status before the surgical procedure takes place and can be used to predict outcomes and the necessary post-operative goals and rehabilitation. System 100 can also use data from historical database 112 to generate implant health score 300. Historical database 112 provides a basis for algorithm 200 to accurately determine an individual patient's implant health score 300 by using multiple patient data to reduce variability. In addition to the various other input metrics described above, this database can be used to analyze and compare different components such as demographics, lifestyle and medical conditions to acquire a general overview of an individual patient's implant health score 300. Algorithm 200 allows for any discrepancies of an individual patient's data versus the historical database to be examined and further tailored for a full spectrum review in order to ensure the best results and most personalized implant health score. System 100 can utilize HCP input 114 to generate implant health score 300. HCP input 114 can include clinical observations, overall patient health condition and other factors not directly related to the implant condition, but that may help system 100 to determine implant health score 300.

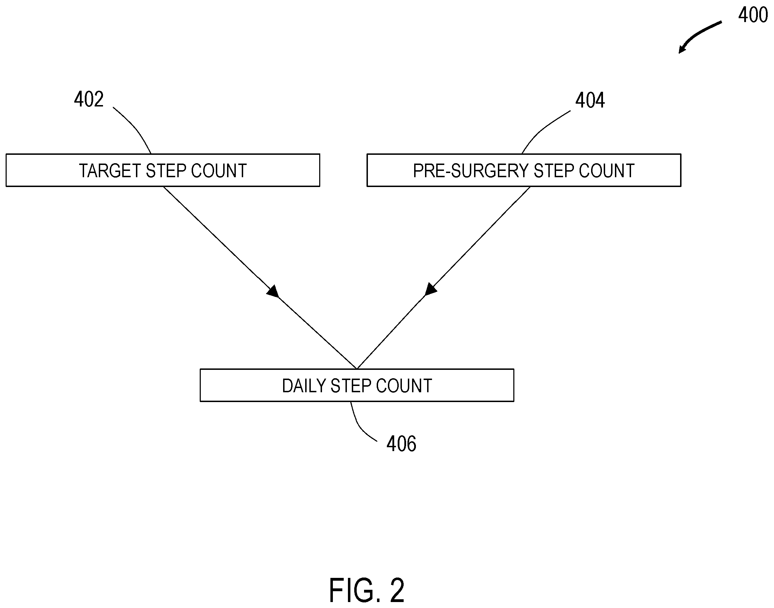

Implant health score 300 can be a simple combined metric such as a weighted sum or mean of a normalized metric values in some embodiments. For example, FIG. 2 shows a schematic view of an implant health score 400 for a joint replacement such as a knee or hip replacement according to an embodiment of the present disclosure. A patient's implant health score 400 can be calculated by first obtaining a daily step count 406 from either the hip joint or knee joint implant. This daily step count 406 is then normalized with the patient's pre-surgery step count 404 or with the target rehabilitation goal step count 402. Depending on the circumstances, one of these two normalizations can be done individually or in conjunction with the other, in order to calculate the implant health score 400. The calculated implant health score 400 for each patient indicates where that individual is in terms of their daily step count 406 in comparison to their pre-surgery target step count 404 and/or target step count 402. A person with a high score will be near the target step count or pre-surgery step count, suggesting that their activity is on track and steady. Conversely, a person with a low score would be below the target step count or pre-surgery step count, indicating that they are not meeting their requirements. Implant health score 400 can be periodically recorded and tracked to assess the patient's rehabilitation process.

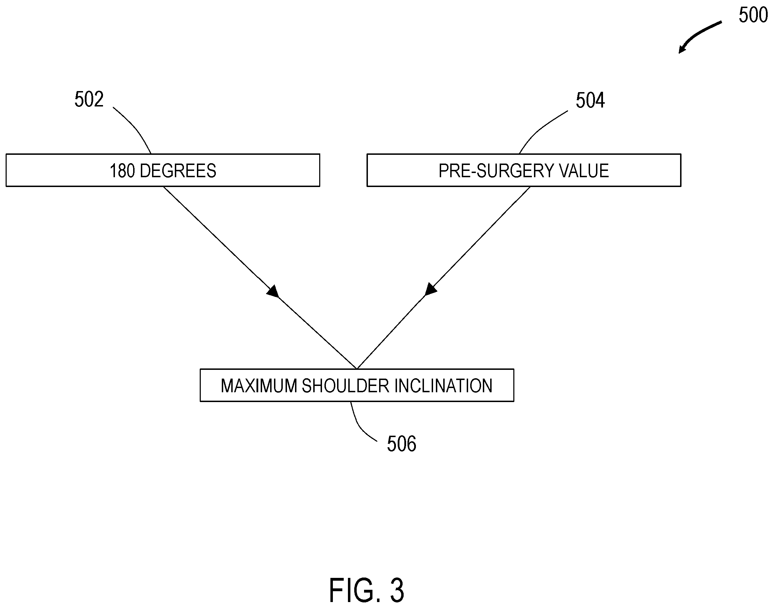

Referring now to FIG. 3, there is shown an implant health score 500 related to shoulder implant performance according to another embodiment of the present disclosure. A patient's maximum shoulder inclination 506 can be normalized by 180 degrees 502 and/or a pre-surgery value 504. In another embodiment, a patient's maximum shoulder inclination 506 can be normalized by subtracting 180 degrees 502 from the original value and/or comparing it to the shoulder's pre-surgery inclination value 504 degrees. This provides an implant health score 500 that allows HCPs to compare the patient's current shoulder inclination to a normalized standard measurement and identify any anomalies.

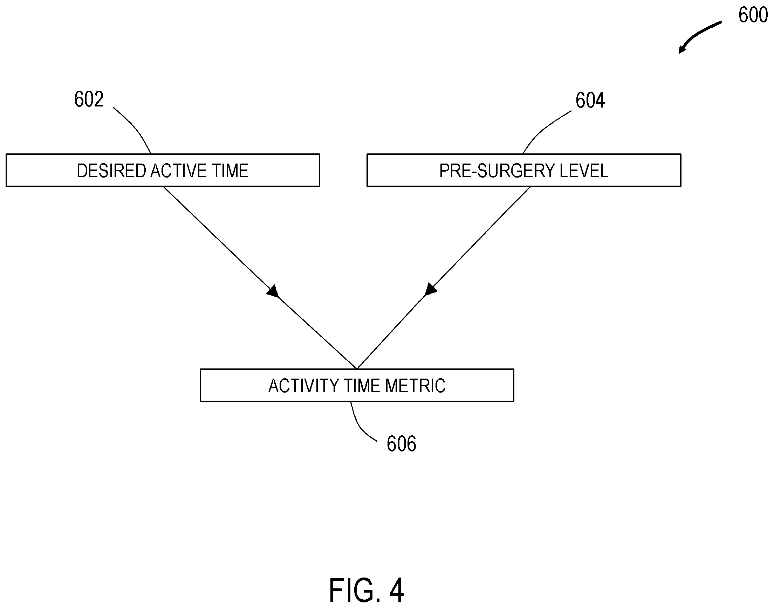

FIG. 4 shows an implant health score 600 associated with patient activity levels according to another embodiment of the present disclosure. For example, a patient activity time metric 606 can be normalized by a desired activity time duration 602 and/or pre-surgery levels 604. Implant health score 600 can be used to measure performance and recovery related to surgery. Implant health score 600 can be used to monitor progress by evaluating patient's activity levels over a defined period of time; outcomes that are significantly different to what was expected by the desired activity time duration 602 and/or pre-surgery levels 604 can be interpreted as indicators of how well the implant is functioning and how well the patient is recovering.

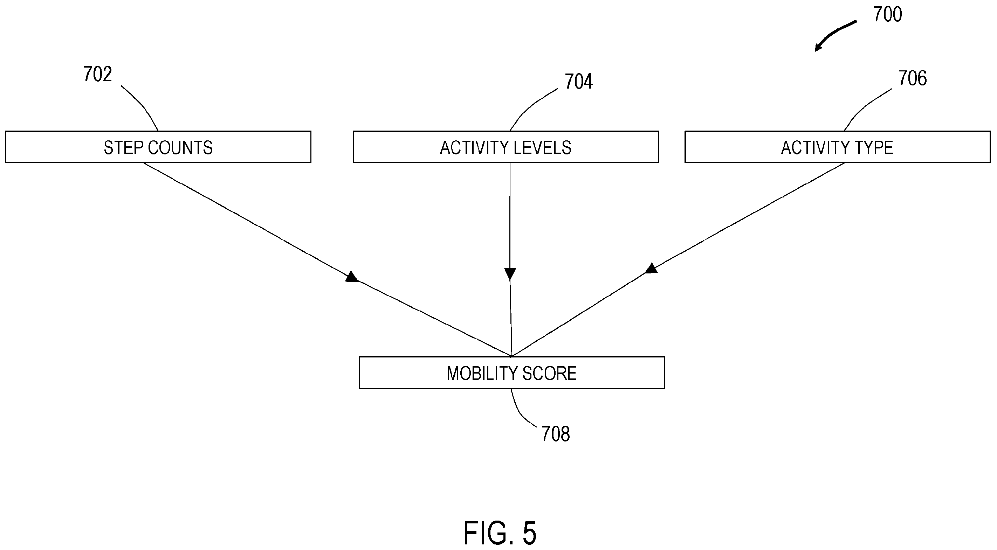

FIG. 5 is a schematic drawing of an implant health score 700 according to another embodiment of the present disclosure. Rather than relying on a single input, the combination of several related metrics can be used to reduce the amount of data provided to the HCP. Averaging techniques can be employed to create a subset of the metrics that can be considered in decision-making, which enables the HCP to more readily identify potential issues with the patient. This would help facilitate the decision-making process and thus improve the care received by the patient. For example, implant health score 700 related to mobility score 708 can be derived from step counts 702, activity levels 704 and activity type 706. Implant health score 700, relating to mobility score 708, can be derived from data collected from sensors implemented into the implant (such as a knee implant) and/or wearable sensors like Inertial Measurement Units (IMU) and heart rate monitors. This data can include step counts 702 that can be tracked by the implant sensors, activity levels 704 that can be tracked by the implant sensors or any external wearables, and activity type 706 tracked by the user via a smartphone or wearable device such as a smartwatch or smartphone. All the data is interpreted by system 100 to produce implant health score 700 that reflects on the patient's mobility after surgery.

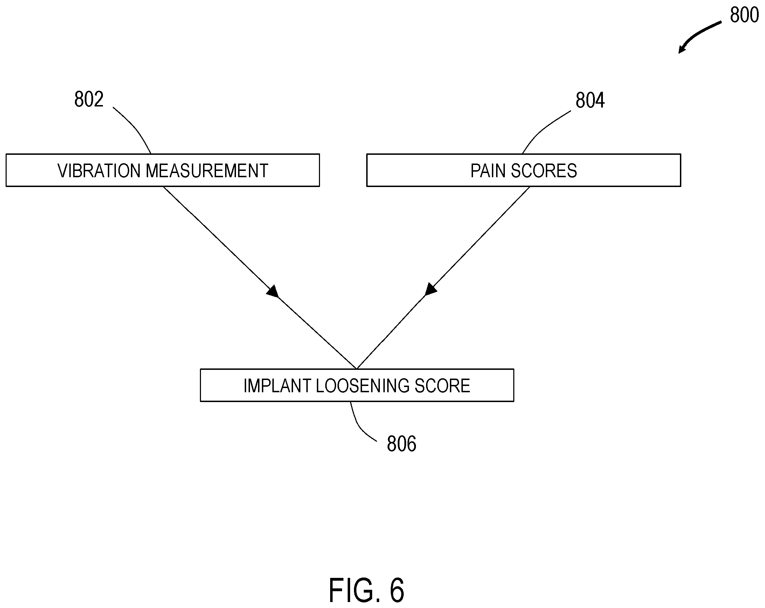

A multiple input implant health score 800 in accordance with the present invention related to implant loosening score 806 is shown in FIG. 6. Here, implant vibration measurement 802 and pain scores 804 are evaluated to generate an implant loosening score. An IMU or other sensor on the joint implant is used to determine implant vibrations, and patient reported pain scores via a patient's smartphone or smart watch is transmitted to system 100 to generate implant loosening score 806. If the implant is loosened, there will be an increase in vibrations and a rise in the patient's reported pain scores; however, if the implant is healing properly, the opposite of this will be observed in the measurements and reports.

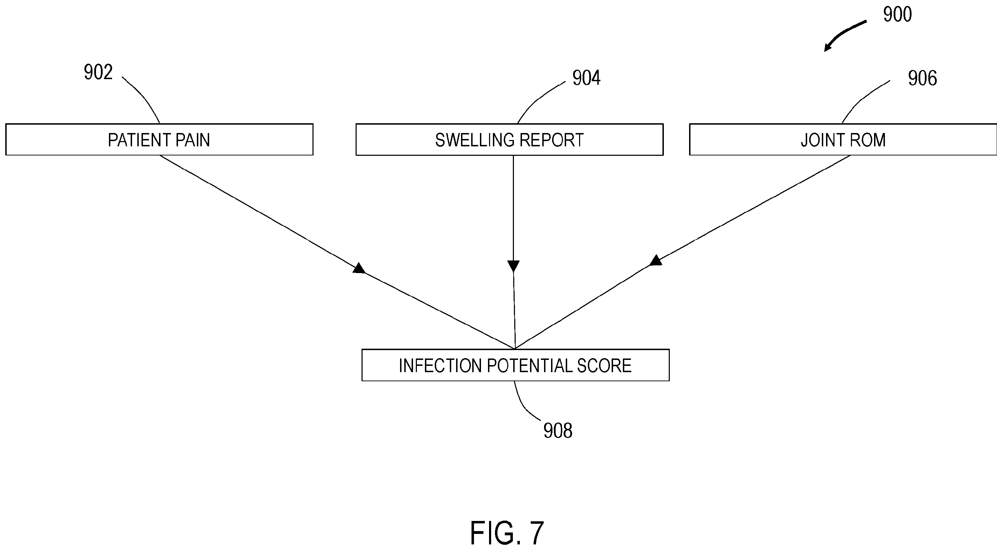

FIG. 7 is a schematic view of an implant health score 900 related to infection detection according to another embodiment of the present disclosure. An infection potential score 908 can be evaluated based on patient pain level 902, swelling reports 904 and joint ROM 906. Infection potential score 908 can be a powerful tool to help HCPs and patients to understand and anticipate the likelihood of an implant-related infection. It takes into account multiple variables, such as patient pain 902 (which can be reported via a patient's smartphone or smartwatch), swelling report 904 (which can be evaluated by an HCP or obtained via a smartphone picture analyzed using machine vision), and joint range of motion (ROM) 906 (which can be determined by an implant's sensors). Additionally, sensors such as pH sensors, temperature sensors, etc. located on the joint implant can also be used to determine infection potential score 908. A historical database with infection potential score limits can be used as reference to properly inform the HCPs and patients about the proper treatment for each infection score in order to avoid unnecessary and costly revision surgeries.

In another embodiment, input metrics can be simplified by using dimensionality reduction techniques, such as principal component analysis (PCA), to recognize redundant metrics. For example, metrics for step counts and active time can sometimes be interchangeable, and PCA would be able to recognize this correlation. By doing so, it helps to limit the amount of data presented to the HCPs by eliminating one of the metrics. This helps to streamline the process, as clinicians are not overwhelmed with an overload of data. In another embodiment, rather than eliminating one of the tightly related metrics, it may be beneficial to take the average of both metrics instead. This could help to provide more comprehensive and accurate data, by preserving both data sets and combining them together.

In another embodiment, implant health scores can be determined by clinical surveys. For example, HCPs can assign numerical rankings or scores to various metrics indicating the degree of importance that each metric has for the individual HCP. Furthermore, the numerical scores would be averaged for each metric and then combined with other metrics to generate a total score, which could be to create an overall weighted average of these metrics. This implant health score could then be utilized to assess the success of an implant by comparing it against the predetermined target values.

Algorithm 200 can utilize AI or machine learning to generate implant health scores. For example, neural networks, regression models, classifiers, and other algorithms can be used to assign scores to patient recovery or assign predictions of patient outcomes. A rating, such as a score from 1 to 10, can be given to a large group of patients by HCPs or the patients themselves in one embodiment. To determine what the score may be in the future, a machine learning model can be used to process data from implant sensors or from other sources to forecast the score. This approach can be used to predict scores that may be reported after an extended period of recovery or to anticipate the score of each patient on any given day without the need for manual input from patients. In another embodiment, models which are capable of collecting, analyzing, and synthesizing data from different types of implant sensors can be employed to predict the likelihood of a patient experiencing an adverse outcome, such as infection, manipulation under anesthesia, or revision surgery. The models can incorporate a variety of factors, including demographics, health history, behavior, and environmental sensors to determine the risk of an adverse outcome. Using this information, the model outputs a score which allows HCPs to quickly assess the patient's likelihood of experiencing one of these adverse outcomes. The HCPs can then use this score to provide personalized, targeted treatment for the patient.

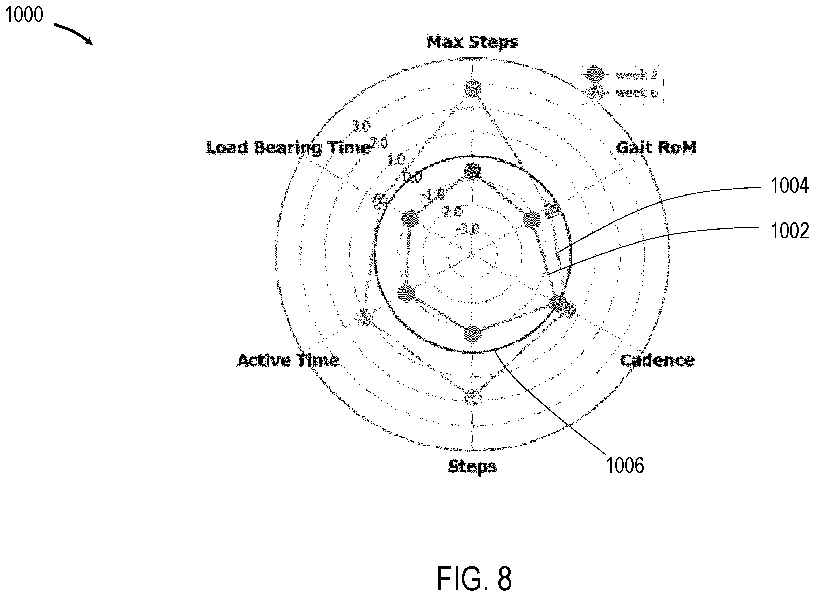

FIG. 8 shows a radar plot 1000 depicting implant health scores according to another embodiment of the present disclosure. Radar plot 1000 can be used to visualize how a patient is recovering and the implant condition at any given moment in time. Radar plot 1000 is comprised of various axis that represent different and independent parameters which indicate the patient's recovery and implant condition. For each axis, a 100% line 1006 represents the target point which is specific to the individual patient. Any score that is below the average is represented by a value that is below 100%, and scores that exceed the average are displayed as a score that is higher than 100%. All the scores of the independent parameters are averaged together in order to make a single conclusion about the overall recovery progress. The area encompassed by the radar plot is a graphical representation of the patient's current recovery status. For example, implant health score 1002 for week 2 after surgery covers a smaller area than implant health score 1004 for week 6 indicating progress in the patient rehabilitation. Using radar plot 1000 to track a patient's progress, it is possible to gain an accurate measurement of their improvement in several individual categories such as activity time, load bearing time, cadence, gait range of motion (ROM), maximum steps and more. This makes it easier to track the patient's progress as they work towards their rehabilitation goals. By monitoring these metrics, HCPs can quickly monitor their patient's progress and make adjustments to their treatment plans as necessary.

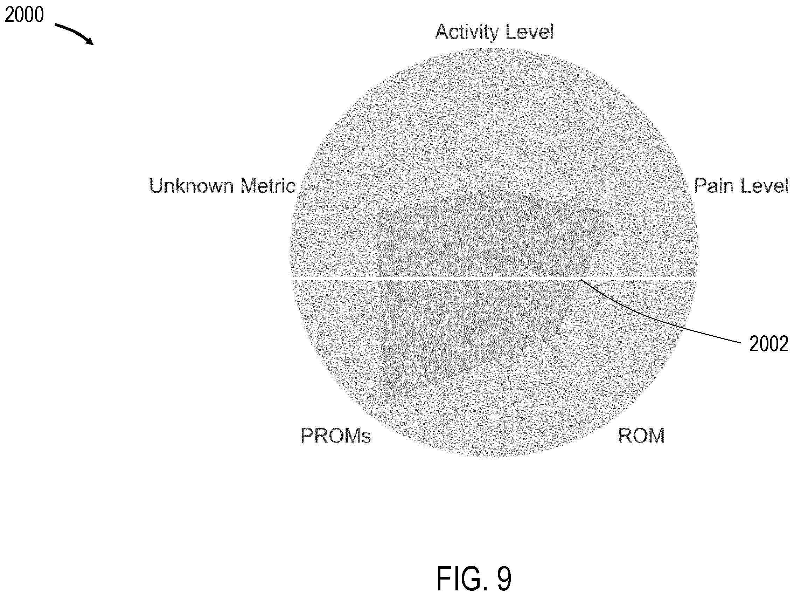

FIG. 9 shows a radar plot 2000 representing an implant health score 2002 for joint replacement surgery according to another embodiment of the present disclosure. Radar plot 2000 can utilize four different axes: pain level, wound healing, joint function, and daily activity level. This allows for quick and effective clinical intervention whenever a patient lags behind in any of these areas. For example, if the pain axis is indicating excessively high pain scores, a prescription for pain medication may be issued. The joint function and activity axis may feature metrics such as cadence while walking, range of motion, or any other relevant indicators, whilst the wound healing axis can be quantified using wound photographs, assessing for presence/absence of redness, stitches, drainage, and the like. In this way, a comprehensive view of the patient's progress can be obtained. The target for each axis must fit the specific needs of each patient, while also accommodating any changes that may occur over the duration of time since the surgery. HCPs can be provided with a set of guidelines to help them decipher the data obtained from the patient's sensors. These instructions are designed to automatically adjust any targets for axis as needed over time, eliminating the need for surgeons to adjust them manually.

While the disclosure herein generally discusses embodiments directed to joint implants with sensors, the components and features disclosed herein are not limited to joint implants but can be used in any other implant or trial with sensors. Sensor shape, size and configuration can be customized based on the type of implant and patient-specific needs.

Furthermore, although the invention disclosed herein has been described with reference to particular features, it is to be understood that these features are merely illustrative of the principles and applications of the present invention. The present invention is defined in the appended claims.

## Claims

1. A computer implemented method for determining an implant condition, the method comprising the steps of:
receiving implant vibration data including vibration measurement (802) from an implant coupled to a bone of a patient, the implant vibration data being generated by a sensor associated with the implant;
receiving patient reported data including pain level (804) from the patient, and
generating, using an algorithm, an implant loosening score (806) from the implant vibration data and the patient reported data,
wherein the implant loosening score increases when the vibration measurement (802) and the patient reported pain level (802) increase, and wherein the implant loosening score (806) is related to movement of implant with respect to the bone.

2. The method of claim 1, wherein the step of generating the implant loosening score includes generating the implant loosening score based on pre-surgery data of the patient.

3. The method of claim 1, wherein the step of generating the implant loosening score includes generating the implant loosening score based on a database of implant loosening scores of multiple patients.

4. The method of claim 1, wherein the implant is a joint implant.

5. The method of claim 4, wherein the sensor is any of an inertial measurement unit sensor, accelerometer, gyroscope, Hall sensor, pH sensor, a temperature sensor and a pressure sensor operatively coupled to a processor of the joint implant.

6. The method of claim 5, wherein the implant includes a plurality of sensors.

7. The method of claim 4, wherein the joint implant is a knee joint implant.

8. The method of claim 7, wherein the patient reported data includes pain level associated with the knee joint implant.

9. The method of claim 8, wherein the implant loosening score is a single numerical value.

10. The method of claim 8, wherein the implant loosening score includes multiple numerical values.

11. The method of claim 8, wherein the implant loosening score is generated as a graphical plot.

12. The method of claim 1, further including a step of receiving input from a healthcare professional and generating the implant loosening score based on the input.

13. The method of claim 12, wherein the joint is any of a knee joint, shoulder joint, and ankle joint.

14. The method of claim 1, wherein the patient reported data includes knee joint swelling data.

15. The method of claim 1, further including the steps of receiving data from a second sensor of the implant and generating an implant infection potential score from the data from the second sensor.

## Patentansprüche

1. Computerimplementiertes Verfahren zum Bestimmen eines Implantatzustands, wobei das Verfahren die folgenden Schritte umfasst:
Empfangen von Implantatvibrationsdaten einschließlich Vibrationsmessung (802) von einem Implantat, das an einen Knochen eines Patienten gekoppelt ist, wobei die Implantatvibrationsdaten durch einen Sensor erzeugt werden, der dem Implantat zugeordnet ist;
Empfangen von patientenberichteten Daten einschließlich Schmerzniveau (804) von dem Patienten, und
Erzeugen, unter Verwendung eines Algorithmus, einer Implantatlockerungsbewertung (806) aus den Implantatvibrationsdaten und den patientenberichteten Daten,
wobei die Implantatlockerungsbewertung zunimmt, wenn die Vibrationsmessung (802) und das patientenberichtete Schmerzniveau (802) zunehmen, und wobei die Implantatlockerungsbewertung (806) mit der Bewegung des Implantats in Bezug auf den Knochen in Beziehung steht.

2. Verfahren nach Anspruch 1, wobei der Schritt des Erzeugens der Implantatlockerungsbewertung das Erzeugen der Implantatlockerungsbewertung basierend auf präoperativen Daten des Patienten beinhaltet.

3. Verfahren nach Anspruch 1, wobei der Schritt des Erzeugens der Implantatlockerungsbewertung das Erzeugen der Implantatlockerungsbewertung basierend auf einer Datenbank von Implantatlockerungsbewertungen von mehreren Patienten beinhaltet.

4. Verfahren nach Anspruch 1, wobei das Implantat ein Gelenkimplantat ist.

5. Verfahren nach Anspruch 4, wobei der Sensor einer von einem Trägheitsmesseinheit-Sensor, Beschleunigungsmesser, Gyroskop, Hall-Sensor, pH-Sensor, einem Temperatursensor und einem Drucksensor ist, der betriebsfähig an einen Prozessor des Gelenkimplantats gekoppelt ist.

6. Verfahren nach Anspruch 5, wobei das Implantat eine Vielzahl von Sensoren beinhaltet.

7. Verfahren nach Anspruch 4, wobei das Gelenkimplantat ein Kniegelenkimplantat ist.

8. Verfahren nach Anspruch 7, wobei die patientenberichteten Daten das mit dem Kniegelenkimplantat assoziierte Schmerzniveau beinhalten.

9. Verfahren nach Anspruch 8, wobei die Implantatlockerungsbewertung ein einzelner numerischer Wert ist.

10. Verfahren nach Anspruch 8, wobei die Implantatlockerungsbewertung mehrere numerische Werte beinhaltet.

11. Verfahren nach Anspruch 8, wobei die Implantatlockerungsbewertung als grafische Darstellung erzeugt wird.

12. Verfahren nach Anspruch 1, ferner beinhaltend einen Schritt des Empfangens einer Eingabe von einem Gesundheitsfachmann und Erzeugen der Implantatlockerungsbewertung basierend auf der Eingabe.

13. Verfahren nach Anspruch 12, wobei das Gelenk eines von einem Kniegelenk, Schultergelenk und Knöchelgelenk ist.

14. Verfahren nach Anspruch 1, wobei die patientenberichteten Daten Kniegelenkschwellungsdaten beinhalten.

15. Verfahren nach Anspruch 1, ferner beinhaltend die Schritte des Empfangens von Daten von einem zweiten Sensor des Implantats und Erzeugen einer Implantatinfektionspotentialbewertung aus den Daten von dem zweiten Sensor.

## Revendications

1. Procédé mis en œuvre par ordinateur pour déterminer l'état d'un implant, le procédé comprenant les étapes suivantes:
recevoir des données de vibration d'un implant comprenant une mesure de vibration (802) d'un implant couplé à un os d'un patient, les données de vibration de l'implant étant générées par un capteur associé à l'implant;
recevoir des données transmises par le patient comprenant un niveau de douleur (804) du patient, et
générer, à l'aide d'un algorithme, un score de détachement de l'implant (806) à partir des données de vibration de l'implant et des données transmises par le patient,
dans lequel le score de détachement de l'implant augmente lorsque la mesure de vibration (802) et le niveau de douleur (802) transmis par le patient augmentent, et dans lequel le score de détachement de l'implant (806) est lié au mouvement de l'implant par rapport à l'os.

2. Procédé selon la revendication 1, dans lequel l'étape de génération du score de détachement de l'implant comprend la génération du score de détachement de l'implant sur la base de données pré-chirurgicales du patient.

3. Procédé selon la revendication 1, dans lequel l'étape de génération du score de détachement de l'implant comprend la génération du score de détachement de l'implant sur la base d'une base de données de scores de détachement de l'implant de plusieurs patients.

4. Procédé selon la revendication 1, dans lequel l'implant est un implant articulaire.

5. Procédé selon la revendication 4, dans lequel le capteur est choisi parmi les types de capteurs suivants: un capteur d'unité de mesure inertielle, un accéléromètre, un gyroscope, un capteur à effet Hall, un capteur de pH, un capteur de température et un capteur de pression couplé de manière opérationnelle à un processeur de l'implant articulaire.

6. Procédé selon la revendication 5, dans lequel l'implant comprend une pluralité de capteurs.

7. Procédé selon la revendication 4, dans lequel l'implant articulaire est un implant articulaire du genou.

8. Procédé selon la revendication 7, dans lequel les données transmises par le patient comprennent un niveau de douleur associé à l'implant articulaire du genou.

9. Procédé selon la revendication 8, dans lequel le score de détachement de l'implant est une valeur numérique unique.

10. Procédé selon la revendication 8, dans lequel le score de détachement de l'implant comprend plusieurs valeurs numériques.

11. Procédé selon la revendication 8, dans lequel le score de détachement de l'implant est généré sous la forme d'un graphique.

12. Procédé selon la revendication 1, comprenant en outre une étape consistant à recevoir des informations d'un professionnel de la santé et à générer le score de détachement de l'implant sur la base de ces informations.

13. Procédé selon la revendication 12, dans lequel l'articulation est l'une des articulations suivantes: une articulation du genou, une articulation de l'épaule et une articulation de la cheville.

14. Procédé selon la revendication 1, dans lequel les données transmises par le patient comprennent des données de gonflement de l'articulation du genou.

15. Procédé selon la revendication 1, comprenant en outre les étapes consistant à recevoir des données d'un second capteur de l'implant et à générer un score d'infection potentielle de l'implant à partir des données du second capteur.
